(19) Europäisches Patentamt
European Patent Office
Office européen des brevets

(11)  **EP 2 081 505 B1**

(12)  # EUROPÄISCHE PATENTSCHRIFT

(45) Veröffentlichungstag und Bekanntmachung des Hinweises auf die Patenterteilung:
**11.12.2013  Patentblatt 2013/50**

(21) Anmeldenummer: **07819288.7**

(22) Anmeldetag: **24.10.2007**

(51) Int Cl.:
***A61B 17/15*** (2006.01)   ***A61B 17/14*** (2006.01)
***A61B 17/17*** (2006.01)

(86) Internationale Anmeldenummer:
**PCT/EP2007/009236**

(87) Internationale Veröffentlichungsnummer:
**WO 2008/049604 (02.05.2008 Gazette 2008/18)**

(54)  **SCHABLONE ZUM DURCHFÜHREN EINER UMSTELLUNGSOSTEOTOMIE**

JIG FOR PERFORMING A TRANSPOSITION OSTEOTOMY

GABARIT PERMETTANT D'EFFECTUER UNE OSTÉOTOMIE DE MODIFICATION

(84) Benannte Vertragsstaaten:
**AT BE BG CH CY CZ DE DK EE ES FI FR GB GR HU IE IS IT LI LT LU LV MC MT NL PL PT RO SE SI SK TR**

(30) Priorität: **25.10.2006  DE 102006050311**

(43) Veröffentlichungstag der Anmeldung:
**29.07.2009  Patentblatt 2009/31**

(73) Patentinhaber: **Brehm, Peter**
**91085 Weisendorf (DE)**

(72) Erfinder:
• **BREHM, Peter**
**91085 Weisendorf (DE)**
• **LIEBEL, Georg**
**90559 Burgthann (DE)**

(74) Vertreter: **Grünecker, Kinkeldey,**
**Stockmair & Schwanhäusser**
**Leopoldstrasse 4**
**80802 München (DE)**

(56) Entgegenhaltungen:
EP-A- 1 302 167    US-A- 5 540 695
US-A- 5 722 978    US-A- 5 980 526

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

**Beschreibung**

Die vorliegende Erfindung bezieht sich allgemein auf orthopädische Instrumente, insbesondere auf eine Schablone zum Durchführen einer Umstellungsosteotomie und eine Vorrichtung zum Durchführen einer Umstellungsosteotomie, die eine derartige Schablone umfasst.

Deformitäten der Stütz- und Bewegungsorgane eines Patienten könnten zu einer Einschränkung der Beweglichkeit des Patienten führen und dadurch dessen Lebensqualität erheblich beeinträchtigen. Außerdem können Fehlstellungen zu einer einseitigen Belastung von Gelenken führen: Dies kann zum vorzeitigen Verschleiß des Gelenks und damit zu Schmerzen und einer Einschränkung der Funktion des Gelenks bis hin zur vollständigen Unbeweglichkeit führen. In manchen Fällen ist es deshalb angezeigt, die Deformität durch eine Operation zu korrigieren, um die Lebensqualität des Patienten zu verbessern und den Verschleiß des Gelenks zu verlangsamen.

Durch eine Fehlstellung der Beinachse kann es zu einer ungleichen Belastung des äußeren und des inneren Anteils des Kniegelenks kommen. Bei einem O-Bein (Genu varum) wird der innere Gelenkspalt stärker belastet als der äußere Gelenkspalt. Umgekehrt kommt es bei einem X-Bein (Genu valgum) zu einer verstärkten Belastung des äußeren Gelenkspalts. In dem stärker belasteten Gelenkspalt kann eine Gonarthrose, d.h. eine Degeneration des Knorpelgewebes mit sekundärer Knochenläsion und entzündlich bedingter Schrumpfung der Gelenkkapsel, auftreten.

Um das Risiko degenerativer Erkrankungen zu verringern, wird in manchen Fällen eine Umstellungsosteotomie durchgeführt. Dabei wird aus dem Schienbein (Tibia) des Patienten im Bereich des Tibiakopfes ein keilförmiges Knochenstück entnommen. Anschließend werden die beiden Schnittflächen des Knochens zusammengebracht und in dieser Stellung fixiert, z.B. mit Hilfe von Metallimplantaten, die mit dem Schienbeinknochen verschraubt werden. Zur Korrektur eines X-Beins kann das keilförmige Knochenstück an der Innenseite des Tibiakopfes entnommen werden. Bei der Korrektur eines O-Beins kann das keilförmige Knochenstück an der Außenseite entnommen werden. Alternativ kann die Umstellungsosteotomie auch am Oberschenkelknochen des Patienten erfolgen.

Neben Umstellungsosteotomien im Bereich des Kniegelenks werden Umstellungsosteotomien im Bereich des Hüftgelenks vorgenommen, um das Hüftgelenk bei Vorliegen einer Koxarthrose, einem Hüftgelenksverschleiß, der durch unterschiedliche mechanische und biologische Faktoren verursacht werden kann, zu zentrieren. Dadurch kann eine gleichmäßigere Verteilung mechanischer Belastungen auf den gesamten Knorpel erreicht werden, was dazu beitragen kann, den weiteren Verschleiß des Hüftgelenks zu verzögern oder gar aufzuhalten. Zu diesem Zweck kann insbesondere, je nach der Art der Fehlstellung im Bereich des Hüftgelenks, eine Varisationsosteotomie oder eine Valgisationsosteotomie durchgeführt werden. Dabei wird aus dem proximalen Bereich des Femur (Oberschenkelknochen) ein keilförmiges Knochenstück entnommen. Bei der Varisationsosteotomie erfolgt die Entnahme des Knochenstücks auf der Innenseite, bei der Valgisationsosteotomie auf der Außenseite.

Entscheidend für den Erfolg der Umstellungsosteotomie ist, dass der Winkel, der zwischen den Teilen des Knochens auf beiden Seiten des entnommenen keilförmigen Knochenstücks eingestellt wird, derart angepasst ist, dass sich eine möglichst günstige Stellung des Gelenks ergibt. Da die Form, in der der Knochen nach der Umstellungsosteotomie zusammenwächst, wesentlich von der Gestalt des entnommenen Knochenstücks beeinflusst wird, muss diese bei der Operation möglichst exakt eingestellt werden.

Üblicherweise erfolgt die Entnahme des keilförmigen Knochenstücks, indem der Knochen des Patienten mit einer Säge geschnitten wird. Wird die Säge dabei von Hand geführt, muss der Operateur viel Erfahrung und ein gutes Augenmaß haben, um die erforderliche Präzision zu erreichen.

Um die Genauigkeit zu erhöhen, können unter Röntgenkontrolle Drahtstifte entsprechend der geplanten Sägeschnitte eingebracht werden, entlang denen dann die oszillierende Säge geführt wird.

Um die Genauigkeit der Umstellungsosteotomie weiter zu verbessern, wurden Navigationstechniken zum Führen der Knochensäge vorgeschlagen. Bei einem Navigationsverfahren nach dem Stand der Technik werden zunächst Schrauben oder Nägel als Navigationshilfen in den Knochen eingesetzt. Längsrichtungen der Navigationshilfen entsprechen dabei der Richtung der Schnitte, die bei der Entnahme des keilförmigen Knochenstücks durchgeführt werden. Nachdem die Navigationshilfen eingesetzt wurden, kann ihre Lage kontrolliert werden, z.B. mit Hilfe einer Röntgenaufnahme. Ergibt die Kontrolle, dass sich die Navigationshilfen an der richtigen Stelle befinden, wird der Knochen entlang der Navigationshilfen gesägt. Stellt man dagegen fest, dass die Navigationshilfen nicht richtig positioniert sind, werden sie entfernt und an einer korrigierten Position erneut eingesetzt.

US 5,722,978 (Basis für den Oberbegriff des Anspruchs 1) offenbart eine Osteotomieführung zum Durchführen einer Tibiaosteotomie. Die Osteotomieführung umfasst zwei oder mehr Reihen von Führungslöchern, die sich durch die Osteotomieführung erstrecken und zum Befestigen der Osteotomieführung auf einem Paar von Führungsstiften, die in einer vorbestimmten Anordnung relativ zum Tibia angeordnet sind, geeignet sind. Außerdem umfasst die Osteotomieführung einen querverlaufenden Schlitz, der eine querverlaufende Schnittfläche definiert, und mehrere schräge Schlitze, die gegen den querverlaufenden Schlitz geneigt sind, wobei jeder geneigte Schlitz eine schräge Schnittebene definiert.

US 5,540,694 offenbart eine Schnittführung zum Durchführen einer Osteotomie. Die Schnittführung umfasst einen Winkelschneidblock, der abnehmbar und justierbar an einer Stabilsierungsbasis befestigt ist.

DE 10 2004 063 977 A1 offenbart eine Bänderspannvorrichtung und eine Schnittlehre zur Femurresektion zur Vorbereitung eines Gelenks für die Implantation eines Gelenkimplantats.

EP 0 366 488 B1 offenbart eine Schnittführung zur Sägeblattführung beim Präparieren eines Oberschenkelknochens für die Implantation einer femoralen Knieprothese.

DE 102 58 322 B3 offenbart eine Führungseinrichtung für ein chirurgisches Bearbeitungswerkzeug.

Ein Nachteil der bisher bei der Umstellungsosteotomie angewandten Techniken liegt darin, dass bei der Entnahme des keilförmigen Knochenstücks mit Hilfe einer von Hand geführten Säge nur eine begrenzte Genauigkeit erreicht werden kann. Außerdem besteht wegen der hohen Anforderungen an den Operator ein großes Fehlerrisiko. Werden Navigationshilfen nach dem Stand der Technik eingesetzt, ist ein gewisser Zeitaufwand erforderlich, um zunächst die Navigationshilfen einzusetzen und die Präzision der Positionierung der Navigationshilfen zu kontrollieren. Außerdem kann das Einsetzen der Navigationshilfen zu Beschädigungen des Knochens führen, was dessen Stabilität verschlechtern kann.

Es ist eine Aufgabe der vorliegenden Erfindung, eine Vorrichtung bereitzustellen, die es ermöglicht, eine präzise Umstellungsosteotomie durchzuführen, wobei die oben erwähnten Nachteile zumindest teilweise vermieden werden können.

Erfindungsgemäß wird diese Aufgabe durch eine Schablone zum Durchführen einer Umstellungsosteotomie mit den Merkmalen des Patentanspruchs 1 gelöst. Ausführungsformen der Erfindung sind in den Patentansprüchen 2 bis 13 definiert.

Die Schablone zum Durchführen einer Umstellungsosteotomie umfasst einen Körper mit mehreren Schlitzen. Jeder Schlitz verläuft entlang einer Längsrichtung und erstreckt sich in einer Tiefenrichtung durch den Körper hindurch. Die Längsrichtungen der Schlitze sind zueinander parallel und die Tiefenrichtungen mindestens eines Paars der Schlitze sind zueinander geneigt.

Bei einer Umstellungsosteotomie kann die Schablone an dem Knochen, aus dem ein keilförmiges Stück zu entnehmen ist, angebracht werden. Ein Instrument zum Durchtrennen eines Knochens, z.B. eine Säge, kann durch einen der Schlitze geführt werden, um einen ersten Schnitt durch den Knochen zu machen. Anschließend kann das Instrument durch einen anderen Schlitz geführt werden, um einen zweiten Schnitt durch den Knochen zu machen. Dadurch kann ein keilförmiges Knochenstück ausgeschnitten werden. Richtungen, in denen die Schnitte verlaufen, und damit eine Form des ausgeschnittenen Knochenstücks, werden dabei im wesentlichen durch die Anordnung der Schlitze in der Schablone und die Auswahl der Schlitze, durch die das Instrument geführt wird, festgelegt. Insbesondere bestimmt die geneigte Anordnung des mindestens einen Paars der Schlitze die Keilform des Knochenstücks. Dadurch kann eine gewünschte Form des entnommenen Knochenstücks mit großer Genauigkeit erreicht werden.

Die Schablone umfasst zusätzlich eine Befestigungsvorrichtung, die zum Befestigen der Schablone an einem Knochen ausgelegt ist. Dadurch kann die Gefahr eines Verrutschens der Schablone während der Entnahme des keilförmigen Knochenstücks und damit das Risiko falsch positionierter Schnitte verringert werden.

Die Befestigungsvorrichtung umfasst einen Befestigungsabschnitt, der eine Bohrung aufweist, durch die ein Befestigungselement, z.B. eine Knochenschraube oder ein Knochennagel, steckbar ist. Ein Verbindungsabschnitt ist mit dem Befestigungsabschnitt und/oder dem Körper drehbar verbunden. Der Befestigungsabschnitt kann mit Hilfe eines durch die Bohrung gesteckten Befestigungselements an dem Knochen fixiert werden. Die drehbare Verbindung zwischen dem Verbindungsabschnitt und dem Befestigungsabschnitt bzw. dem Körper ermöglicht eine Anpassung der Position, in der die Schablone an den Knochen angelegt wird, an die individuelle Anatomie des Patienten.

Geeigneterweise ist der Verbindungsabschnitt relativ zum Befestigungsabschnitt um eine erste Achse drehbar. Der Körper kann relativ zum Verbindungsabschnitt um eine zweite Achse, die zu der ersten Achse parallel ist, drehbar sein. Durch Drehungen um die beiden Achsen kann der Körper relativ zum Befestigungsabschnitt innerhalb einer Ebene bewegt werden, deren Normalenrichtung zu der ersten Achse und der zweiten Achse parallel ist.

In manchen Ausführungsformen der Erfindung ist der Körper relativ zum Verbindungsabschnitt in einer zur ersten Achse senkrechten Längsrichtung des Verbindungsabschnitts verschiebbar. Dadurch kann ein Abstand zwischen dem Befestigungselement und dem Körper an die Anatomie des Patienten angepasst werden.

Der Körper kann eine oder mehrere Bohrungen umfassen. Durch die Bohrungen können Knochennägel und/oder Knochenschrauben geführt werden, mit deren Hilfe der Körper am Knochen des Patienten befestigt werden kann. Damit wird neben der Befestigung mit Hilfe des Befestigungsabschnitts eine weitere Möglichkeit geschaffen, den Körper der Schablone am Knochen zu fixieren. Nach dem Befestigen des Körpers am Knochen ist die relative Lage von Knochen und Schablone im wesentlichen fixiert, so dass im wesentlichen keine weiteren relativen Verschiebungen mehr auftreten können.

Die Tiefenrichtungen jedes Paars der Schlitze können zueinander geneigt sein. Durch Auswahl von zwei zueinander geneigten Schlitzen, durch die das Instrument zum

Durchtrennen des Knochens geführt wird, können unterschiedliche Abmessungen des ausgeschnittenen Knochenkeils eingestellt werden.

Die Tiefenrichtungen des mindestens einen Paars der Schlitze können radial zu einer Schnittlinie verlaufen.

Dadurch treffen sich Schnitte, die mit Hilfe eines durch die Schlitze geführten Instruments durchgeführt werden, an der Schnittlinie. Eine Länge des entfernten keilförmigen Knochenstücks wird somit durch den Abstand zwischen dem Körper und der Schnittlinie bestimmt.

Die Tiefenrichtungen aller Schlitze können radial zu der Schnittlinie verlaufen. Damit ist die Länge des entfernten keilförmigen Knochenstücks im wesentlichen unabhängig von der gewählten Kombination von Schlitzen. Der Keilwinkel des Knochenstücks kann so unabhängig von der Länge des Knochenstücks eingestellt werden.

Erfindungsgemäß umfasst eine Vorrichtung zum Durchführen einer Umstellungsosteotomie eine Schablone wie oben beschrieben und ein Sägeblatt, das durch die Schlitze der Schablone steckbar ist. Das Sägeblatt kann so durch die Schlitze der Schablone präzise geführt werden.

Die Vorrichtung kann zusätzlich eine Antriebseinheit umfassen, die dafür ausgelegt ist, das Sägeblatt zu bewegen. Dadurch kann eine für die Operation benötigte Zeitdauer im Vergleich zu Ausführungsformen, in denen das Sägeblatt dafür ausgelegt ist, von Hand bewegt zu werden, verringert werden.

Die Antriebseinheit kann dafür ausgelegt sein, das Sägeblatt hin und her zu bewegen. Durch eine hin- und hergerichtete Bewegung des Sägeblatts können Verletzungen elastischer Gewebe des Patienten, die der Bewegung des Sägeblatts folgen können, vermieden werden. Die starren Knochen folgen der Bewegung des Sägeblatts nicht, so dass sie vom Sägeblatt geschnitten werden.

Ausführungsformen der Erfindung werden mit Bezug auf die beigefügten Figuren erläutert. Es zeigen:

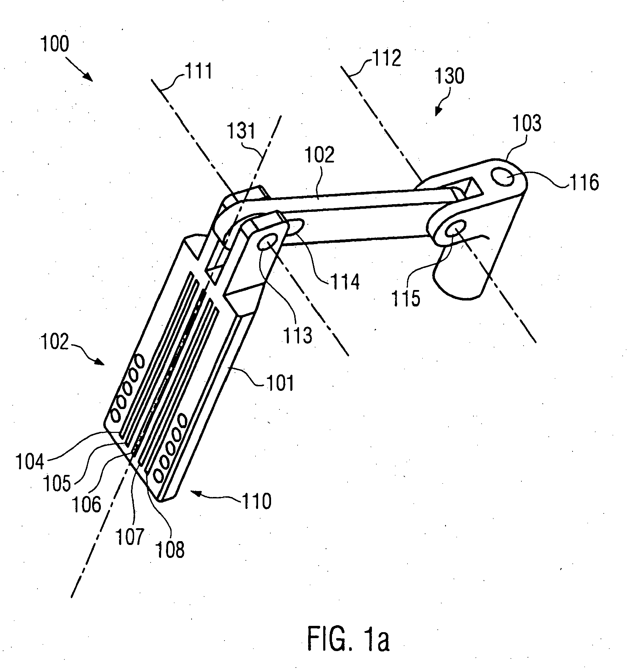

Fig. 1a eine schematische Perspektivansicht einer Schablone zum Durchführen einer Umstellungsosteotomie gemäß einer Ausführungsform der vorliegenden Erfindung;

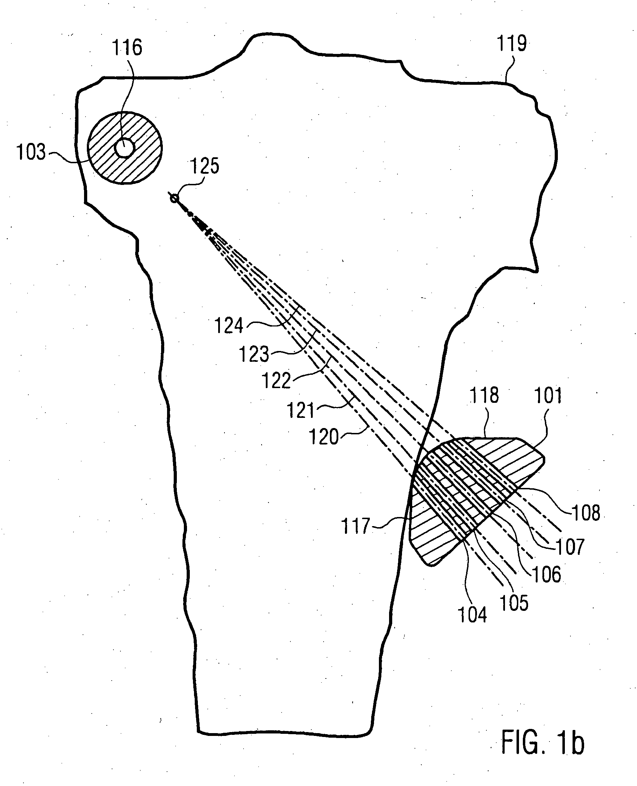

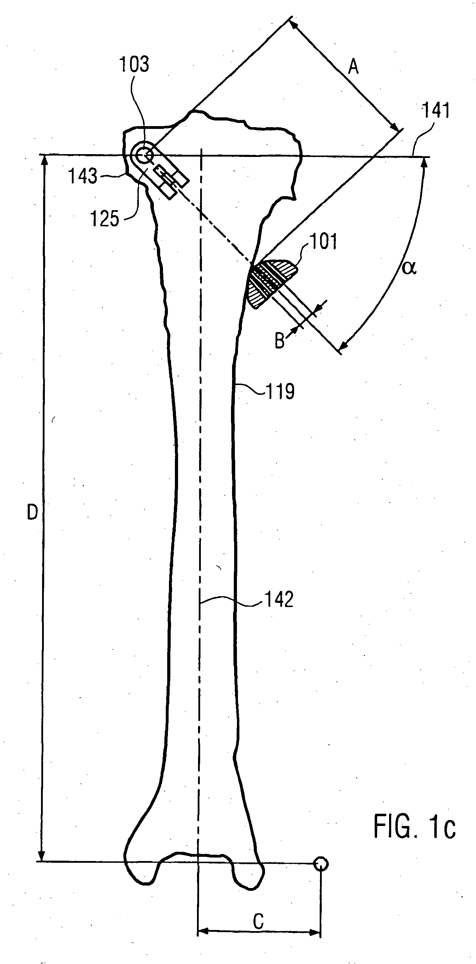

Fig. 1 b eine schematische Querschnittsansicht der in Fig. 1a gezeigten Schablone;

Fig. 1 c eine schematische Skizze, die die geometrischen Verhältnisse bei einer Umstellungsosteotomie veranschaulicht, die mit Hilfe der in den Fig. 1a und 1 b gezeigten Schablone durchgeführt wird; und

Fig. 2 eine schematische Draufsicht eines Sägeblatts in einer Vorrichtung zum Durchführen einer Umstellungsosteotomie gemäß einer Ausführungsform der vorliegenden Erfindung.

Fig. 1a zeigt eine schematische Perspektivansicht einer Schablone 100 zum Durchführen einer Umstellungsosteotomie gemäß einer Ausführungsform der vorliegenden Erfindung. Eine schematische Querschnittsansicht der Schablone 100 ist in Fig. 1b gezeigt. Die Schablone 100 umfasst einen Körper 101 und eine Befestigungsvorrichtung 130, die zum Befestigen der Schablone 100 an einem Knochen 119 (Fig. 1 b) geeignet ist. Der Knochen 119 kann beispielsweise ein Schienbein oder ein Oberschenkelknochen sein.

In dem Körper 101 sind mehrere Schlitze 104, 105, 106, 107, 108 vorgesehen. Eine Längsrichtung von jedem der Schlitze 104-108 verläuft parallel zu einer Längsachse 131 des Körpers 101. Somit sind die Längsrichtungen der Schlitze 104-108 zueinander parallel. Die Schlitze 104-108 erstrecken sich in einer Tiefenrichtung durch den Körper 101 hindurch. Die Tiefenrichtung des Schlitzes 104 verläuft dabei entlang einer Linie 120. Entsprechend verläuft eine Tiefenrichtung des Schlitzes 105 entlang einer Linie 121. Die Linien 122, 123, 124 bezeichnen Tiefenrichtungen der Schlitze 106, 107, 108.

Die Schlitze 104-108 verlaufen radial zu einer Schnittlinie 125, die parallel zur Längsachse 131 des Körpers 101 und senkrecht zu der Zeichenebene der Fig. 1b verläuft. Somit liegt ein Schnittpunkt der Linien 120-124 auf der Schnittlinie 125. Die Tiefenrichtungen jedes Paars der Schlitze 104-108 sind in der in Fig. 1a und 1b gezeigten Ausführungsform zueinander geneigt. Ein Paar der Schlitze 104, 108 kann dabei aus einem beliebigen der Schlitze 104-108 und einem anderen der Schlitze 104-108 gebildet sein. Beispielsweise kann ein Paar durch die Schlitze 104 und 108 gebildet sein und ein anderes Paar kann durch die Schlitze 106 und 107 gebildet sein. Die Tiefenrichtung des Schlitzes 104 ist dabei zu der Tiefenrichtung des Schlitzes 108 geneigt und die Tiefenrichtung des Schlitzes 106 ist zu der Tiefenrichtung des Schlitzes 107 geneigt. In manchen Ausführungsformen können die Tiefenrichtungen jedes beliebigen Paars der Schlitze 104-108 miteinander einen spitzen Winkel einschließen.

Die vorliegende Erfindung ist nicht auf Ausführungsformen beschränkt, in denen die Tiefenrichtungen jedes beliebigen Paars der Schlitze zueinander geneigt sind. In anderen Ausführungsformen können die Tiefenrichtungen von zwei oder mehr der Schlitze 104-108 zueinander parallel sein. Beispielsweise können in einer Ausführungsform der vorliegenden Erfindung die Tiefenrichtungen der Schlitze 104 und 105 zueinander parallel sein und die Tiefenrichtungen der Schlitze 107 und 108 können zueinander parallel sein. Dabei können die Tiefenrichtungen eines Paars von Schlitzen, das einen der Schlitze 104, 105 und einen der Schlitze 107, 108 umfasst, zueinander geneigt sein. Daraus folgt, dass die Schnittlinien unterschiedlicher Paare der Schlitze 104-108 sich in unterschiedlichen Abständen von dem Körper 101 der Schablone 100 befinden. Damit lässt sich durch die Wahl geeigneter Paare der Schlitze 104 - 108 nicht nur der gewünschte Winkel für die Umstellungsosteotomie bestimmen, sondern auch noch die Dicke des Knochens mitberücksichtigen.

Grundsätzlich können die Schlitze 104 - 108 im Körper 101 der Schablone 100 so angeordnet sein, dass die Tiefenrichtungen jeweiliger Paare von Schlitzen jeweils unterschiedliche eingeschlossene Winkel und/oder unterschiedliche Entfernungen der Schnittlinien ihrer Schnittebenen vom Schablonenkörper 101 besitzen.

Der Körper 101 der Schablone 100 weist an der Position der Schlitze 104 - 108 Markierungen auf, die die gegenseitige Neigung zusammengehöriger Paare von Schlitzen anzeigen. Es können auch für jeden Schlitz individuelle Markierungen vorhanden sein, die zusammen mit einer die gegenseitigen Schlitzneigungen berücksichtigenden Tabelle für jedes Paar der Schlitze 104 - 108 die gegenseitige Neigung und den Abstand ihrer Schnittlinien vom Schablonenkörper 101 wiedergeben.

Die Befestigungsvorrichtung 130 umfasst einen Befestigungsabschnitt 103 und einen Verbindungsabschnitt 102. Der Befestigungsabschnitt 103 weist eine Bohrung 116 auf. Ein Durchmesser der Bohrung 116 ist derart ausgelegt, dass ein Befestigungselement eines den Fachleuten bekannten Typs durch die Bohrung 116 gesteckt werden kann. Das Befestigungselement kann dabei insbesondere eine Knochenschraube oder einen Knochennagel umfassen. Ein Kopf des Befestigungselements kann einen Durchmesser haben, der größer als der Durchmesser der Bohrung 116 ist, so dass das in den Knochen 119 eingebrachte Befestigungselement den Befestigungsabschnitt 103 am Knochen 119 festhält. In manchen Ausführungsformen der vorliegenden Erfindung ist das Befestigungselement ohne Kopf ausgeführt. So kann die Befestigungsvorrichtung vorteilhafterweise aufgesteckt und abgenommen werden.

In manchen Ausführungsformen der vorliegenden Erfindung, in der das Befestigungselement eine Knochenschraube umfasst, kann die Bohrung 116 ein Innengewinde aufweisen, das ein Gegengewinde zu einem Gewinde des Befestigungselements bildet. Dadurch kann eine besonders stabile Verbindung zwischen dem Befestigungsabschnitt 103 und dem Befestigungselement sowie dem Knochen 119 geschaffen werden. In anderen Ausführungsformen kann eine Innenfläche der Bohrung 116 glatt sein. Dadurch kann eine Drehbarkeit des Befestigungsabschnitts 103 relativ zum Knochen 119 auch bei eingesetztem Befestigungselement erreicht werden. Vorteilhafterweise erhält man dadurch einen zusätzlichen Freiheitsgrad, der eine größere Flexibilität der Ausrichtung der Schablone 100 relativ zum Knochen 119 ermöglicht.

Der Verbindungsabschnitt 102 kann drehbar mit dem Befestigungsabschnitt 103 verbunden sein, wobei der Verbindungsabschnitt 102 um eine erste Achse 112 drehbar ist. Zu diesem Zweck kann der Verbindungsabschnitt 102 eine Bohrung aufweisen, durch die ein Bolzen 115 gesteckt ist, der mit dem Befestigungsabschnitt 103 verbunden ist, wobei eine Längsachse des Bolzens 115 mit der ersten Achse 112 zusammenfällt.

Zwischen dem Verbindungsabschnitt 102 und dem Körper 101 kann ebenfalls eine drehbare Verbindung vorgesehen sein, wobei der Körper 101 relativ zum Verbindungsabschnitt 102 um eine zweite Achse 111 drehbar ist. Die zweite Achse 111 kann dabei parallel zu der ersten Achse 112 sein. Zur drehbaren Verbindung mit dem Körper 101 kann der Verbindungsabschnitt 102 eine Öffnung 114 aufweisen, durch die ein mit dem Körper 101 verbundener Bolzen 113 gesteckt wird.

Die Öffnung 114 kann die Form eines Schlitzes haben, der sich entlang einer Längsrichtung des Verbindungsabschnitts 102 erstreckt, wobei die Längsrichtung senkrecht zu der ersten Achse 112 sein kann. Der Bolzen 113 und der mit diesem verbundene Körper 101 können so entlang der Längsrichtung des Verbindungsabschnitts 102 verschoben werden. Ferner kann der Körper 101 um die zweite Achse 111 gedreht werden, wobei sich die zweite Achse bei einer Verschiebung des Körpers 101 entlang der Längsrichtung des Verbindungsabschnitts 102 mitbewegt. Die zweite Achse 111 kann bei der Verschiebung im wesentlichen parallel zu der ersten Achse 112 bleiben.

Bei einer Verschiebung des Körpers 101 entlang der Längsrichtung des Verbindungselements 102 und einer Drehung des Körpers 101 um eine oder beide der Achsen 112, 111 bewegt sich der Körper 101 in einer Ebene, deren Normalenrichtung parallel zu den Achsen 111, 112 ist.

Der Körper 101 muss nicht entlang der Längsrichtung des Verbindungsabschnitts 102 verschiebbar sein. In anderen Ausführungsformen der vorliegenden Erfindung kann die Öffnung 114 in Form einer im wesentlichen runden Bohrung bereitgestellt werden, in der der Bolzen 113 gedreht, aber im wesentlichen nicht verschoben werden kann.

In dem Körper 101 können Bohrungen 109, 110 vorgesehen sein, wobei eine erste Gruppe 109 von Bohrungen auf einer anderen Seite der Schlitze 104-108 vorgesehen ist als eine zweite Gruppe 110 von Bohrungen. Die Bohrungen 109, 110 sind dafür geeignet, Knochennägel und/oder Knochenschrauben hindurch zu stecken, um den Körper 101 der Schablone 100 am Knochen 119 zu befestigen. Dies kann - neben der Befestigung des Befestigungsabschnitts 103 am Knochen 119 - dazu beitragen, ein Verrutschen des Körpers 101 zu verhindern.

Auf einer der Schnittlinie 125 zugewandten Seite kann der Körper 101 Abschrägungen 117, 118 aufweisen. Durch die Abschrägungen 117, 118 kann eine größere Auflagefläche des Körpers 101 auf dem Knochen 119 erreicht werden, zumindest aber ein näheres Heranführen der Schablone an den Knochen. Dadurch können ein stabilerer Sitz des Körpers 101 auf dem Knochen 119 und eine Verteilung von Druckkräften, die der Körper 101 auf den Knochen 119 ausübt, auf eine größere Kontaktfläche erreicht werden.

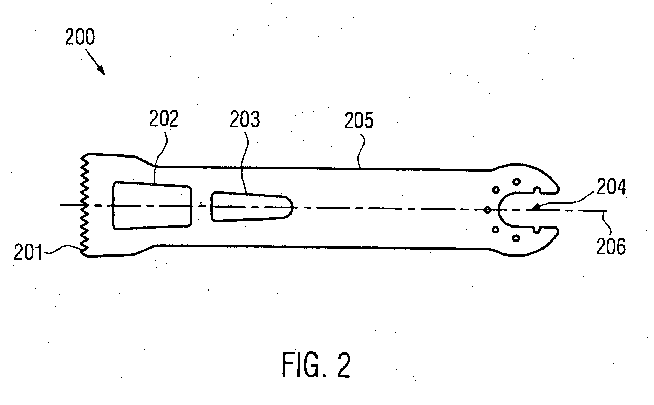

Eine Vorrichtung zum Durchführen einer Umstellungsosteotomie gemäß einer Ausführungsform der vorliegenden Erfindung kann neben der Schablone 100 ein Sägeblatt umfassen, das durch die Schlitze 104-108 steckbar ist. Fig. 2 zeigt eine schematische Draufsicht eines Sägeblatts 200, das in einer Vorrichtung gemäß der vorliegenden Erfindung verwendet werden kann.

Das Sägeblatt 200 umfasst eine Platte 205. Die Platte 205 kann eine längliche Form haben, wobei eine Ausdehnung der Platte 205 in einer Längsrichtung (in Fig. 2 durch eine gestreichelte Linie 206 dargestellt) größer als eine Ausdehnung der Platte 205 in einer zu der Längsrichtung 206 senkrechten Querrichtung sein kann. An einem Ende der Platte 205 sind Sägezähne 201 angebracht. Eine Form der Sägezähne 201 kann denen einer den Fachleuten bekannten Knochensäge entsprechen. Die Sägezähne können entlang der Querrichtung der Platte 205 angeordnet sein. Im Inneren der Platte 205 können Öffnungen 202, 203 angebracht sein. Dadurch wird eine Gewichts- und Materialersparnis sowie eine bessere Sicht des Operateurs auf das Operationsfeld ermöglicht.

An einem den Sägezähnen 201 gegenüber liegenden Ende des Sägeblatts 205 ist eine Einspannvorrichtung 204 vorgesehen, die dafür ausgelegt ist, in eine Antriebseinheit eines den Fachleuten bekannten Typs eingespannt zu werden. Die Antriebseinheit kann dafür ausgelegt sein, das Sägeblatt 200 entlang der Querrichtung der Platte 205 zu bewegen, so dass die Sägezähne 201 Späne des Knochens 119 abtragen können. Die Antriebseinheit kann dafür ausgelegt sein, das Sägeblatt in einer Schwingbewegung hin-und herzubewegen, wobei eine Amplitude der Schwingung derart ausgelegt sein kann, dass elastisches Gewebe wie zum Beispiel menschliche Haut der Bewegung der Sägezähne 201 folgen kann. Dadurch können Verletzungen des Patienten und/oder des Operateurs bei einer unbeabsichtigten Berührung des Sägeblatts 200 vermieden werden.

Zum Durchführen einer Umstellungsosteotomie mit Hilfe der oben beschriebenen Vorrichtung wird zunächst mit den Fachleuten bekannten Methoden ein Zugang zu dem Knochen 119 des Patienten geschaffen. Der Knochen 119 kann beispielsweise ein Schienbein (Tibia) oder ein Oberschenkelknochen (Femur) oder ein anderer Knochen sein. Anschließend wird der Befestigungsabschnitt 103 der Schablone 100 an dem Knochen 119 befestigt. Zu diesem Zweck kann eine Knochenschraube oder ein Knochennagel durch die Bohrung 116 des Befestigungsabschnitts 103 geführt und in den Knochen 119 geschraubt bzw. eingeschlagen werden.

Fig. 1c zeigt eine schematische Skizze, die die Befestigung der Schablone 100 an dem Knochen 119 darstellt, wobei der Knochen 119 beispielhaft ein Schienbein des Patienten darstellt. Der Befestigungsabschnitt 103 wird dabei an der Tibia 143 befestigt. Dabei wird zwischen einer Richtung 141, die zu einer Längsrichtung 142 des Knochens 119 senkrecht ist, und der Längsrichtung des Verbindungsabschnitts 102 der Schablone 100 ein vorbestimmter Winkel $\alpha$ eingestellt. Der Winkel $\alpha$ kann beispielsweise einen Wert im Bereich von 30° bis 60° insbesondere einen Wert von ungefähr 45° haben. Durch Drehen des Verbindungsabschnitts 102 und des Körpers 101 um die erste Achse 112 und die zweite Achse 111 sowie durch Verschieben des Körpers 101 entlang der Längsrichtung des Verbindungsabschnitts 102 kann anschließend eine Position des Körpers 101 so eingestellt werden, dass der Körper 101 auf dem Knochen 119 aufliegt.

Anschließend kann der Körper 101 durch Knochennägel und/oder Knochenschrauben, die durch die Bohrungen 109, 110 geführt werden, an dem Knochen 119 fixiert werden.

Entsprechend der zu korrigierenden Fehlstellung des Beins des Patienten wird ein Paar der Schlitze 104-108 ausgewählt. Die Auswahl des Paars der Schlitze 104-108 wird im Folgenden mit Bezug auf Fig. 1 c beschrieben. In Fig. 1c bezeichnet D eine Länge des Knochens 119 und C eine seitliche Verschiebung des distalen Endes des Knochens 119 in einer zur Längsrichtung 142 des Knochens senkrechten Richtung, die durch die Umstellungsosteotomie erreicht werden soll (der eigentliche Korrekturbetrag der Achse). Die Parameter D und C können mit Hilfe den Fachleuten bekannter Verfahren bestimmt werden. Beispielsweise können die Parameter D und C anhand einer Röntgenaufnahme des Patienten ausgemessen werden. A bezeichnet einen Abstand zwischen einem Paar der Schlitze 104-108 und der Schnittlinie 125, an der sich die Tiefenrichtungen des Paars der Schlitze 104-108 schneiden. B bezeichnet den Abstand zwischen den beiden Schlitzen des Paars.

Wenn aus dem Knochen 119 ein keilförmiges Knochenstück entnommen wird, das sich von der Schnittlinie 125 bis zu dem Körper 101 erstreckt und am Körper 101 eine Breite hat, die dem Abstand B zwischen einem Paar der Schlitze 104-108 entspricht, und die beiden an das keilförmige Knochenstück angrenzenden Schnittflächen des Knochens 119 miteinander verbunden werden, verschiebt sich das distale Ende des Knochens 119 um den Abstand C, wenn das Verhältnis zwischen dem Abstand C und der Länge D des Knochens gleich dem Verhältnis zwischen dem Abstand B des Paars der Schlitze 104-108 und dem Abstand A zwischen dem Körper 101 und der Schnittlinie 125 ist. Daraus ergibt sich für den Abstand B der Schlitze:

$$B = \frac{A \cdot C}{D} \qquad\qquad (1)$$

Von den Schlitzen 104-108 werden zwei Schlitze ausgewählt, deren Abstand möglich nahe an dem mit Hilfe der Gleichung (1) bestimmten Abstand B liegt. Um die Auswahl der Schlitze zu vereinfachen, können auf der von der Schnittlinie 125 abgewandten Seite des Körpers 101 Markierungen angebracht sein, die den Abstand bestimmter Paare der Schlitze 104-108 angeben.

Anschließend wird das Sägeblatt 200 durch einen ersten Schlitz des ausgewählten Paars von Schlitzen geführt. Durch den ersten Schlitz wird die Bewegungsfreiheit des Sägeblatts 200 eingeschränkt. Das Sägeblatt kann sich im wesentlichen nur innerhalb einer Ebene, die durch die Längsrichtung des Schlitzes und die Tiefenrichtung des Schlitzes bestimmt wird, bewegen. Wenn das Sägeblatt 200 bewegt wird, beispielsweise mit Hilfe einer mit dem Sägeblatt 200 verbundenen Antriebseinheit, wird der Knochen in der von der Längsrichtung und der Tiefenrichtung des ersten Schlitzes aufgespannten Ebene geschnitten. Insbesondere trifft der Schnitt auf die Schnittlinie 125, an der sich die Tiefenrichtungen der Schlitze 104-108 treffen. Der Sägevorgang wird solange fortgesetzt, bis der Schnitt die Schnittlinie 125 erreicht.

Anschließend wird das Sägeblatt 200 durch den zweiten Schlitz des Paars von Schlitzen geführt und ein Sägevorgang durchgeführt. Durch den zweiten Schlitz wird die Bewegungsfreiheit des Sägeblatts 200 derart eingeschränkt, dass sich das Sägeblatt 200 im wesentlichen nur innerhalb einer von der Längsrichtung und der Querrichtung des zweiten Schlitzes aufgespannten Ebene bewegen kann, so dass der Knochen entlang dieser Ebene geschnitten wird. Dadurch trifft der vom zweiten Schlitz aus geführte Schnitt an der Schnittlinie 125 auf den vom ersten Schlitz aus durchgeführten Schnitt. Sobald sich die beiden Schnitte getroffen haben, kann der Sägevorgang beendet werden. Durch die beiden Schnitte wird aus dem Knochen 119 ein keilförmiges Knochenstück ausgeschnitten, das auf der dem Körper 101 zugewandten Seite eine Breite hat, die im wesentlichen dem Abstand B entspricht, und sich bis zu einer Tiefe in den Knochen 119 hinein erstreckt, die im wesentlichen dem Abstand A entspricht. Die vorliegende Erfindung ermöglicht somit das Ausschneiden eines keilförmigen Knochenstücks mit den gewünschten Abmessungen, wobei die gewünschten Abmessungen des Knochenstücks mit großer Genauigkeit erreicht werden können.

Anschließend kann die Schablone 100 vom Knochen 119 gelöst werden. Zu diesem Zweck werden die Knochenschrauben und/oder Knochennägel, die durch die Bohrung 116 im Befestigungsabschnitt 103 und die Bohrungen 109, 110 im Körper 101 geführt wurden, aus dem Knochen 119 entfernt. Auch das ausgeschnittene keilförmige Knochenstück wird entfernt.

Daraufhin werden die beiden Schnittflächen des Knochens 119 derart ausgerichtet, dass sie miteinander in Kontakt stehen und in dieser Stellung fixiert. Zu diesem Zweck kann an dem Knochen 119 ein Implantat eines den Fachleuten bekannten Typs angebracht werden. Schließlich wird der Zugang zum Körper des Patienten geschlossen und die Haut des Patienten vernäht.

## Patentansprüche

1.  Schablone (100) zum Durchführen einer Umstellungsosteotomie, umfassend:

    einen Körper (101) mit mehreren Schlitzen (104-108), wobei jeder Schlitz entlang einer Längsrichtung verläuft und sich in einer Tiefenrichtung (120-124) durch den Körper hindurch erstreckt, die Längsrichtungen der Schlitze zueinander parallel sind und die Tiefenrichtungen mindestens eines Paars der Schlitze zueinander geneigt sind; und
    eine Befestigungsvorrichtung (130), die zum Befestigen der Schablone an einem Knochen ausgelegt ist; wobei die Befestigungsvorrichtung umfasst:

    einen Befestigungsabschnitt (103) mit einer Bohrung (116), durch die ein Befestigungselement, insbesondere eine Knochenschraube oder ein Knochennagel, steckbar ist; und
    einen Verbindungsabschnitt (102) zwischen dem Befestigungsabschnitt und dem Körper; **dadurch gekennzeichnet dass** der Verbindungsabschnitt mit dem Befestigungsabschnitt und/oder dem Körper drehbar verbunden ist.

2.  Schablone nach Anspruch 1, wobei der Verbindungsabschnitt (102) relativ zum Befestigungsabschnitt um eine erste Achse (112) drehbar ist.

3.  Schablone nach Anspruch 2, wobei der Körper (101) relativ zum Verbindungsabschnitt (102) um eine zweite Achse (111) drehbar ist, die zu der ersten Achse (112) parallel ist.

4.  Schablone nach einem der Ansprüche 2 und 3, wobei der Körper (101) relativ zum Verbindungsabschnitt (102) in einer zur ersten Achse (112) senkrechten Längsrichtung des Verbindungsabschnitts verschiebbar ist.

5.  Schablone nach einem der Ansprüche 1 bis 4, wobei der Körper (101) eine oder mehrere Bohrungen umfasst.

**6.** Schablone nach einem der Ansprüche 1 bis 5, wobei die Tiefenrichtungen (120-124) jedes Paars der Schlitze (104-108) zueinander geneigt sind.

**7.** Schablone nach einem der Ansprüche 1 bis 6, wobei die Tiefenrichtungen (120-124) des mindestens einen Paars der Schlitze (104-108) radial zu einer Schnittlinie verlaufen.

**8.** Schablone nach Anspruch 7, wobei die Tiefenrichtungen (120-124) aller Schlitze (104-108) radial zu der Schnittlinie verlaufen.

**9.** Schablone nach einem der Ansprüche 6 bis 8, wobei der Körper (101) auf einer der Schnittlinie zugewandten Seite mindestens eine Abschrägung (117, 118) aufweist.

**10.** Vorrichtung zum Durchführen einer Umstellungsosteotomie, umfassend:

eine Schablone (100) nach einem der Ansprüche 1 bis 9; und
ein Sägeblatt (200), das durch die Schlitze (104-108) der Schablone steckbar ist.

**11.** Vorrichtung nach Anspruch 10, wobei das Sägeblatt (200) derart durch die Schlitze (104-108) der Schablone (100) steckbar ist, dass Sägezähne (101) des Sägeblatts entlang der Längsrichtung der Schlitze angeordnet sind.

**12.** Vorrichtung nach einem der Ansprüche 10 und 11, zusätzlich umfassend eine Antriebseinheit, die dafür ausgelegt ist, das Sägeblatt (200) zu bewegen.

**13.** Vorrichtung nach Anspruch 12, wobei die Antriebseinheit dafür ausgelegt ist, das Sägeblatt (200) hin und her zu bewegen.

**Claims**

**1.** Guide (100) for performing a transposition osteotomy, comprising:

a body (101) with a plurality of slots (104 - 108), wherein each slot runs along a longitudinal direction and extends through the body in a depth direction (120 - 124), the longitudinal directions of the slots are parallel to one another and the depth directions of at least one pair of the slots are inclined towards one another; and
an attachment device (130) which is designed to attach the guide to a bone;
wherein the attachment device comprises:

an attachment section (103) with a hole (116) through which an attachment element, in particular a bone screw or a bone nail, can be passed; and
a connecting section (102) between the attachment section and the body; **characterised in that** the connecting section is turnably connected to the attachment section and/or the body.

**2.** Guide according to claim 1, wherein the connecting section (102) can be turned relative to the attachment section on a first axis (112).

**3.** Guide according to claim 2, wherein the body (101) can be turned relative to the connecting section (102) on a second axis (111) which is parallel to the first axis (112).

**4.** Guide according to one of claims 2 and 3, wherein the body (101) can be moved relative to the connecting section (102) in a longitudinal direction of the connecting section vertical to the first axis (112).

**5.** Guide according to one of claims 1 to 4, wherein the body (101) comprises one or a plurality of holes.

**6.** Guide according to one of claims 1 to 5, wherein the depth directions (120 - 124) of each pair of slots (104 - 108) are inclined towards one another.

**7.** Guide according to one of claims 1 to 6, wherein the depth directions (120 - 124) of the at least one pair of the slots (104 - 108) run radially to a cutting line.

**8.** Guide according to claim 7, wherein the depth directions (120 - 124) of all slots (104 - 108) run radially to the cutting line.

**9.** Guide according to one of claims 6 to 8, wherein the body (101) has at least one chamfer (117, 118) on a side facing the cutting line.

**10.** Device for performing a transposition osteotomy, comprising:

a guide (100) according to one of claims 1 to 9; and
a saw blade (200) which can be passed through the slots (104 - 108) of the guide.

**11.** Device according to claim 10, wherein the saw blade (200) can be passed through the slots (104 - 108) of the guide (100) such that saw teeth ($101_{[A1]}$) of the saw blade are arranged along the longitudinal direction of the slots.

**12.** Device according to one of claims 10 and 11, additionally comprising a drive unit which is designed to move the saw blade (200).

**13.** Device according to claim 12, wherein the drive unit is designed to move the saw blade (200) back and forth.

## Revendications

**1.** Gabarit (100) destiné à pratiquer une ostéotomie corrective d'adaptation, comprenant :

un corps (101) présentant plusieurs fentes (104-108), chaque fente s'étendant le long d'une direction longitudinale et s'étendant à travers le corps selon une direction de profondeur (120-124), les directions longitudinales des fentes étant parallèles les unes aux autres et les directions de profondeur d'au moins une paire des fentes étant inclinées les unes par rapport aux autres ; et
un dispositif de fixation (130), qui est conçu pour fixer le gabarit sur un os ;
le dispositif de fixation comprenant :

un tronçon de fixation (103) avec un alésage (116) à travers lequel il est possible d'engager un élément de fixation, notamment une vis d'ostéosynthèse ou un clou ou une broche d'ostéosynthèse ; et
un tronçon de liaison (102) entre le tronçon de fixation et le corps ;

**caractérisé en ce que** le tronçon de liaison est relié de manière rotative au tronçon de fixation et/ou au corps.

**2.** Gabarit selon la revendication 1, dans lequel le tronçon de liaison (102) peut tourner par rapport au tronçon de fixation, autour d'un premier axe (112).

**3.** Gabarit selon la revendication 2, dans lequel le corps (101) peut tourner par rapport au tronçon de liaison (102), autour d'un deuxième axe (111), qui est parallèle au premier axe (112).

**4.** Gabarit selon l'une des revendications 2 et 3, dans lequel le corps (101) peut coulisser par rapport au tronçon de liaison (102), dans une direction longitudinale du tronçon de liaison, perpendiculaire au premier axe (112).

**5.** Gabarit selon l'une des revendications 1 à 4, dans lequel le corps (101) comprend un ou plusieurs alésages.

**6.** Gabarit selon l'une des revendications 1 à 5, dans lequel les directions de profondeur (120-124) de chacune des paires de fentes (104-108), sont inclinées les unes par rapport aux autres.

**7.** Gabarit selon l'une des revendications 1 à 6, dans lequel les directions de profondeur (120-124) de ladite au moins une paire des fentes (104-108) s'étendent radialement par rapport à une ligne de coupe.

**8.** Gabarit selon la revendication 7, dans lequel les directions de profondeur (120-124) de toutes les fentes (104-108) s'étendent radialement par rapport à la ligne de coupe.

**9.** Gabarit selon l'une des revendications 6 à 8, dans lequel le corps (101) présente, sur un côté dirigé vers la ligne de coupe, au moins une partie biseautée (117, 118).

**10.** Dispositif destiné à pratiquer une ostéotomie corrective d'adaptation, comprenant :

    un gabarit (100) selon l'une des revendications 1 à 9 ; et
    une lame de scie (200), qui peut être engagée à travers les fentes (104-108) du gabarit.

**11.** Dispositif selon la revendication 10, dans lequel la lame de scie (200) peut être engagée à travers les fentes (104-108) du gabarit (100) de manière telle, que les dents de scie (201) de la lame de scie soient agencées le long de la direction longitudinale des fentes.

**12.** Dispositif selon l'une des revendications 10 et 11, comprenant en outre une unité d'entraînement, qui est conçue pour déplacer la lame de scie (200).

**13.** Dispositif selon la revendication 12, dans lequel l'unité d'entraînement est conçue pour déplacer la lame de scie (200) en va-et-vient.

FIG. 1b

FIG. 1c

FIG. 2

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 5722978 A
- US 5540694 A
- DE 102004063977 A1
- EP 0366488 B1
- DE 10258322 B3